# EUROPÄISCHE PATENTSCHRIFT

(11) **EP 1 127 545 B1**
(45) Veröffentlichungstag und Bekanntmachung des Hinweises auf die Patenterteilung: **30.05.2007**
(21) Anmeldenummer: 01200562.5
(22) Anmeldetag: 19.02.2001
(51) Int. Cl.: A61B 6/03, A61B 6/12, A61B 19/00

(54) **Verfahren zur Lokalisierung von Objekten in der interventionellen Radiologie**
Procedure for locating objects in radiotherapy
Procédé pour individuer des objets en radiothérapie

(30) Priorität: 26.02.2000 DE 10009166
(43) Veröffentlichungstag der Anmeldung: 29.08.2001
(73) Patentinhaber: Philips Intellectual Property & Standards GmbH, 20099 Hamburg (DE); Koninklijke Philips Electronics N.V., 5621 BA Eindhoven (NL)
(72) Erfinder: Rasche, Volker, Dr., Habsburgerallee 11, 52064 Aachen (DE); Schmitz, Georg, Dr., Habsburgerallee 11, 52064 Aachen (DE); Sabczynski Jörg, Dr., Habsburgerallee 11, 52064 Aachen (DE); Zylka, Waldemar, Dr., Habsburgerallee 11, 52064 Aachen (DE); Van Vaals, Johannes Jacobus, Habsburgerallee 11, 52064 Aachen (DE)
(74) Vertreter: Kopf, Korbinian Paul

(56) Entgegenhaltungen:
- WO-A-99/27839
- WO-A-99/59106
- US-A- 2 464 586
- US-A- 5 240 000
- US-A- 5 772 594
- US-A- 5 799 055
- US-A- 5 836 954
- US-A- 5 852 646
- US-A- 5 872 829
- US-A- 5 951 475
- US-A- 5 963 613

## Beschreibung

Die Erfindung betrifft ein Verfahren zur Lokalisierung eines Objektes, insbesondere in der interventionellen Radiologie, bei dem ein Volumenbereich, in welchem sich das zu lokalisierende Objekt befindet, anhand von Projektionsbildern mittels digitaler Bildverarbeitung untersucht wird. Des weiteren betrifft die Erfindung ein radiologisches Diagnosegerät zur Durchführung des Verfahrens, chirurgische Instrumente, die in der interventionellen Radiologie Verwendung finden und ein Computer-Programmprodukt, das in der interventionellen Radiologie zur digitalen Bildverarbeitung eingesetzt wird.

In der interventionellen Radiologie ist generell die exakte Lokalisierung von bestimmten dreidimensionalen Objekten, wie z. B. Kathetern, Nadeln, Endoskopen etc., von entscheidender Bedeutung. Beispielsweise bei röntgengeführten Biopsien muss ein Volumen von gegebenenfalls weniger als 1 mm³ gezielt mit der Punktionsnadel getroffen werden. In der bildgeführten orthopädischen Chirurgie muss die Positionierung und Orientierung z. B. von Schrauben im Knochengewebe mit hoher Genauigkeit überwacht werden. Bei jeder Art von minimalinvasiven Eingriffen ist der Operateur darauf angewiesen, die räumliche Lage der verwendeten Instrumente relativ zum Patienten präzise bestimmen zu können.

Während eines typischen bildgeführten Eingriffes wird der Patient laufend mittels Röntgendurchstrahlung beobachtet. Dazu wird vorzugsweise ein C-Arm-Röntgengerät verwendet, mit welchem es möglich ist, die räumliche Lage eines chirurgischen Instrumentes dadurch zu bestimmen, dass die Röntgendurchleuchtung in zwei unterschiedlichen, nahezu senkrechten Projektionsrichtungen durchgeführt wird. Dem Operateur stehen beide Ansichten gleichzeitig zur Verfügung, so dass ein dreidimensionaler Eindruck der Position und Orientierung der verwendeten chirurgischen Instrumente im Operationsgebiet gewonnen werden kann. Bei der Überwachung der Operation muss die Durchleuchtung des Patienten in kurzen Zeitabständen wiederholt werden, so dass die Bewegung der chirurgischen Instrumente verfolgt werden kann. Dabei wird gegebenenfalls wiederholt zwischen den beiden Projektionsrichtungen des C-Arm-Durchleuchtungsgerätes gewechselt. Das Verfahren ist aufwendig und langwierig und bedeutet darüber hinaus sowohl für den Patienten als auch für den Operateur eine unangemessen hohe Belastung mit ionisierender Strahlung.

Die US 5 772 594 schlägt ein System für die bildgeführte Chirurgie vor, bei dem der Patient, das Röntgengerät und der außerhalb des Patienten befindliche Teil eines chirurgischen Instrumentes mit Leuchtdioden (LED's) versehen sind. Die relative räumliche Lage von Patient, Röntgengerät und chirurgischem Instrument wird mittels eines optischen Meßsystems ermittelt. Mit Hilfe von Röntgen-opaken Markierungen, die ebenfalls am Patienten angebracht werden, kann die Position des chirurgischen Instrumentes zu den Röntgenbildern in Beziehung gesetzt werden. Ein digitales Bildverarbeitungssystem dient dazu, eine Darstellung der Position und der Orientierung des chirurgischen Instrumentes den verschiedenen Röntgenansichten zu überlagern.

Bei diesem vorbekannten optisch arbeitenden System für die bildgeführte Chirurgie ist nachteilig, dass eine umfangreiche, zusätzliche Hardware-Ausstattung nötig ist. Die Anwendung beschränkt sich auf chirurgische Geräte, die mit Leuchtdioden in geeigneter Weise ausgestattet sind und damit vom Bildverarbeitungssystem lokalisiert werden können. Nachteilig ist des weiteren, dass eine Bewegung des Patienten während der Prozedur ausgeschlossen werden muss, da ansonsten eine korrekte Zuordnung der räumlichen Lage des chirurgischen Instrumentes zu den Röntgenansichten nicht mehr möglich ist. US-5836954 offenbart ein Verfahren gemäß dem Oberbegriff des ersten Anspruchs.

Dementsprechend liegt der vorliegenden Erfindung die Aufgabe zugrunde, ein Verfahren zur Objektlokalisierung bereitzustellen, das ohne zusätzliche Gerätekomponenten auskommt und eine zuverlässige, kontinuierliche und vollautomatische Bestimmung der Position und der Orientierung eines Objektes, insbesondere eines chirurgischen Instrumentes, gestattet. Bei der Anwendung in der interventionellen Radiologie soll dadurch eine Visualisierung der relativen räumlichen Lage eines Objektes relativ zum Patienten ermöglicht werden.

Diese Aufgabe wird bei einem Verfahren der eingangs genannten Art dadurch gelöst, dass Projektionen des zu lokalisierenden Objektes, dessen geometrische Form bekannt ist, simuliert werden und mit dem Projektionsbild des untersuchten Volumenbereiches verglichen werden.

Das erfindungsgemäße Verfahren verwendet zweidimensionale Projektionsbilder, wie sie z. B. mittels Röntgendurchleuchtung erzeugt werden, um daraus die Objektposition und - orientierung zu bestimmen. Die digitale Bildverarbeitung des zweidimensionalen Datensatzes stellt vergleichsweise niedrige Ansprüche an die Leistungsfähigkeit der verwendeten Datenverarbeitungsanlage. Das erfindungsgemäße Verfahren lässt sich somit auf nahezu jedem Computersystem, das zur Bildverarbeitung in radiologischen Diagnosegeräten standardmäßig eingesetzt wird, implementieren. Es handelt sich also um eine reine Software-Erweiterung von bereits vorhandenen radiologischen Diagnosegeräten, die mit einem entsprechend niedrigen Kostenaufwand jederzeit bereitgestellt werden kann.

Bei den allermeisten Anwendungen in der interventionellen Radiologie sind Objekte zu lokalisieren, deren geometrische Form bekannt ist. Wird ein dreidimensionales geometrisches Modell - z. B. eines chirurgischen Instrumentes - der digitalen Bildverarbeitung zur Verfügung gestellt, so besteht die Möglichkeit, zweidimensionale Projektionsbilder daraus zu berechnen. Dieser Simulation ist neben den geometrischen Objektdaten ein jeweils gerätespezifisches Modell der Bildentstehung zugrundezulegen (Kameramodell). Durch Vergleich der berechneten Projektionen mit dem realen Projektionsbild ist es dann auf einfache Weise möglich, die gewünschte Objektposition und -orientierung zu ermitteln.

Das erfindungsgemäße Verfahren benötigt prinzipiell zur Objektlokalisierung lediglich ein einzelnes zweidimensionales Bild. Damit entfällt grundsätzlich die Notwendigkeit, zur dreidimensionalen Objektlokalisierung zwischen verschiedenen Projektionsrichtungen wechseln zu müssen. Die dreidimensionale Information steht jederzeit zur Verfügung, da die Verarbeitung der vergleichsweise geringen Datenmenge des zweidimensionalen Bilddatensatzes in kürzester Zeit erfolgen kann.

Werden bei der Lokalisierung jedoch mehrere reale Projektionsbilder, nach Möglichkeit mit jeweils unterschiedlichen Projektionsrichtungen, verwendet, lässt sich die Zuverlässigkeit und die Genauigkeit des Verfahrens noch erheblich steigern. Die Verwendung von zwei oder mehr Projektionsbildern ist insbesondere dann vorteilhaft, wenn die zweidimensionale Projektion des zu lokalisierenden Objektes nicht eindeutig auf die Position und die Orientierung zurückschließen lässt.

Die Simulation der Projektionen wird ausgehend von den sechs Objektfreiheitsgraden (Position und Orientierung) durchgeführt. Aufgrund der geringen Zahl der Parameter, die in die Simulation eingehen, ist das erfindungsgemäße Lokalisierungsverfahren extrem schnell bei niedrigen Anforderungen an die Computer-Hardware, sehr robust und einfach zu implementieren.

Zweckmäßigerweise wird die Objektlokalisierung derart ausgeführt, dass unter systematischer Variation der Objektfreiheitsgrade die Simulation iterativ solange wiederholt wird, bis die Abweichungen zwischen berechneter Simulation und realem Projektionsbild minimal sind. Dabei kann die Prozedur abgebrochen werden, sobald die Objektposition und die Objektorientierung mit der vorgegebenen Genauigkeit ermittelt sind. Zur Bewertung der Übereinstimmung der simulierten Projektion mit dem realen Bild werden an sich bekannte Bildverarbeitungs- und erkennungsalgorithmen verwendet.

Das erfindungsgemäße Verfahren kann Vorteilhafterweise dadurch weiter vereinfacht werden, dass das zu lokalisierende Objekt mit einer oder mehreren Markierungen versehen wird, die dann auf dem Projektionsbild abgebildet werden. Die Markierungen können dabei eine sehr einfache geometrische Form haben, die mit minimalem Aufwand dem Bildverarbeitungssystem zur Verfügung gestellt werden kann. Die Simulation der Projektionen derartiger Markierungen gestaltet sich noch erheblich einfacher, als die Berechnung von gegebenenfalls komplexen dreidimensionalen Objekten. Dadurch lässt sich das Lokalisierungsverfahren noch schneller und effektiver durchführen. Aus der Kenntnis der Anordnung der Markierungen relativ zu dem zu lokalisierenden Objekt wird dann die Bestimmung der gewünschten Objektposition und -orientierung durchgeführt.

Da das erfindungsgemäße Lokalisierungsverfahren ein zweidimensionales Projektionsbild zur Bestimmung der Objektlage verwendet, sind die genannten Markierungen derartig zu gestalten, dass deren Projektionsbild einen eindeutigen Rückschluss auf die Position und die Orientierung zulässt. Hierzu eignen sich insbesondere Markierungen aus linienartigen, sich schneidenden Strukturen, wobei die Linienabschnitte durch den Schnittpunkt in Strecken mit jeweils unterschiedlichen Längenverhältnissen unterteilt werden. Durch die Größe und die Lage des Projektionsbildes der Markierung kann auf die Objektposition zurückgeschlossen werden; durch die unterschiedlichen Längenverhältnisse der Markierungsabschnitte wird jeder beliebigen Objektorientierung eindeutig ein Projektionsbild zugeordnet.

Zur Bestimmung der räumlichen Lage und Orientierung eines Objektes mit dem erfindungsgemäßen Verfahren kann Vorteilhafterweise ein dreidimensionales Volumenmodell benutzt werden, welches mittels dreidimensionaler Kernspinresonanz oder Computertomographie gewonnen wird. Im klinischen Umfeld, in dem das Verfahren gemäß der Erfindung zum Einsatz kommt, besteht in der Regel auch die Möglichkeit, dreidimensionale Volumenabbildungen mit den genannten Methoden aufzunehmen. Die damit erhaltenen geometrischen Daten von zu lokalisierenden Objekten, seien es chirurgische Instrumente oder auch Knochen oder innere Organe eines Patienten, können dann erfindungsgemäß als Ausgangspunkt für die Simulation von Projektionsbildern dienen. Auch hierbei können die zu lokalisierenden Objekte mit den obengenannten Markierungen versehen sein. Die Volumendaten können dann dazu benutzt werden, die Anordnung der Markierungen an dem zu lokalisierenden Objekt zu bestimmen.

Zur Durchführung des erfindungsgemäßen Lokalisierungsverfahrens eignet sich ein radiologisches Diagnosegerät mit einem Patiententisch, einem digitalen Bilderzeugungssystem, einem Computer und wenigstens einem Anzeigegerät, bei dem im Arbeitsspeicher des Computers ein gerätespezifisches, mathematisches Modell der Bildentstehung, geometrische Daten von zu lokalisierenden Objekten und ein Lokalisierungsprogramm abgelegt sind, das anhand der Geräte- und Objektdaten die Parameter Objektposition und Objektorientierung relativ zum Patiententisch aus dem Projektionsbild ermittelt und auf dem Anzeigegerät visualisiert.

Radiologische Diagnosegeräte mit der bezeichneten Hardware-Ausstattung finden standardmäßig in der interventionellen Radiologie Verwendung. Das erfindungsgemäße Lokalisierungsverfahren wird auf dem für die Bildverarbeitung eingesetzten Computer implementiert. Dafür wird ein gerätespezifisches Kameramodell benötigt, so dass Prokjektionsbilder ausgehend von den geometrischen Daten der zu lokalisierenden Objekte berechnet werden können. Aus der Übereinstimmung der simulierten Projektionen mit dem realen Projektionsbild wird dann die Objektposition und Objektorientierung relativ zum Patiententisch bestimmt, so dass die vollständige Information über die Objektlage relativ zum Patienten zur Verfügung steht. Ausgehend von den ermittelten Parametern Objektposition und Objektorientierung kann die dreidimensionale Lage des Objektes auf dem Anzeigegerät dargestellt werden, wobei es z. B. möglich ist, dass lokalisierte Objekt in verschiedenen Ansichten realen Projektionsbildern des untersuchten Volumens zu überlagern.

Wie zuvor erwähnt, finden in der interventionellen Radiologie insbesondere C-Arm-Durchleuchtungsgeräte weitverbreitete Anwendung. Bei diesen Geräten wird der Patient von einem konusförmigen Röntgenstrahlungsbündel durchleuchtet, das von einer Röntgenquelle ausgeht, die gegenüber einem fluoroskopischen Bilderzeugungssystem angeordnet ist. Die hierbei entstehenden digitalen Projektionsbilder können unmittelbar für das erfindungsgemäße Lokalisierungsverfahren verwendet werden. Das dafür benötigte Kameramodell umfasst Informationen über den Abstand zwischen Röntgenquelle und Bilderzeugungssystem (Detektor) sowie über den Schnittpunkt der optischen Achse mit der Detektionsebene. Durch diese Parameter wird unter anderem der Vergrößerungsmaßstab bei der Bildaufnahme festgelegt. Dieser muss natürlich bei der dreidimensionalen Lokalisierung berücksichtigt werden.

Der Einsatz des erfindungsgemäßen Verfahrens ist aber auch auf Computer- oder Kernspintomographen ohne weiteres möglich. Bei Computertomographen entstehen üblicherweise entweder Schnittbilder oder dreidimensionale Volumenabbildungen. Bei diesen Geräten kann also ein zusätzlicher Datenverarbeitungsschritt nötig sein, um daraus die benötigten Projektionsbilder zu erzeugen. Bei Kerspintomographen besteht grundsätzlich die Möglichkeit, bei der Bildaufnahme auf die Scheibenselektion zu verzichten bzw. mittels einer geeigneten Kombination von Magnetfeldgradienten mit entsprechenden Hochfrequenzpulsen vergleichsweise dicke Scheiben zu selektieren. Damit ist es dann unmittelbar möglich, statt der sonst üblichen Schnittbilder Projektionsbilder eines vorgegebenen Volumenbereiches in frei wählbaren Projektionsrichtungen zu erhalten. Diese können dann für die Objektlokalisierung gemäß der Erfindung benutzt werden.

Die interventionelle Radiologie in Computer- oder Kernspintomographen findet immer weitere Verbreitung. Derartige Systeme verfügen stets über ein leistungsfähiges Computersystem zur Bildverarbeitung. Das auf Projektionsbildern basierende Lokalisierungsverfahren gemäß der Erfindung eignet sich insbesondere bei diesen Systemen dafür, eine schnelle und zuverlässige Objektlokalisierung durchzuführen, wobei die Möglichkeit besteht, dass lokalisierte Objekt in die verschiedenen Visualisierungsmöglichkeiten, über die diese Systeme verfügen, einzubeziehen.

Chirurgische Instrumente, die sich in Verbindung mit dem erfindungsgemäßen Lokalisierungsverfahren zur Verwendung in der interventionellen Radiologie eignen, verfügen über wenigstens eine Markierung, die geometrisch derart gestaltet ist, dass sich ihrem zweidimensionalen Projektionsbild, eindeutig die Position und die Orientierung der Markierung zuordnen lässt. Besonders vorteilhaft ist eine Markierung, die aus linienartigen, sich schneidenden Strukturen besteht, die an ihrem Schnittpunkt in Abschnitte mit jeweils unterschiedlichen Längenverhältnissen unterteilt werden.

Wie oben beschrieben, haben derartige Markierungen vorteilhafte Eigenschaften, was die Ermittlung der Position und der Orientierung des chirurgischen Instrumentes aus Projektionsbildern angeht. Erfolgt die Bilderzeugung mittels Röntgendurchleuchtung oder mittels Computertomographie, so sind die Markierungen zweckmäßigerweise Röntgenopak oder Röntgensemitransparent. Röntgen-opake Markierungen liefern zwar einen sehr deutlichen Kontrast im Projektionsbild, was für die digitale Bildverarbeitung durchaus vorteilhaft ist. Allerdings haben semitransparente Markierungen den Vorteil, dass durch sie keinerlei Bildelemente verdeckt werden, wodurch unter Umständen für den Chirurgen wichtige Informationen verloren gehen würden. Semitransparente Markierungen haben den weiteren Vorteil, dass sie mittels bekannter Bildverarbeitungstechniken nachträglich aus den Darstellungen entfernt werden können. Ein guter Kompromiss, der die Vorteile von semitransparenten Markierungen und die Anforderungen an die Objektlokalisierung gleichermaßen berücksichtigt, besteht darin, dass die Markierungen aus einer Kombinationen von Röntgen-opaken und -semitransparenten Elementen bestehen.

Chirurgische Instrumente, die bei der interventionellen Radiologie in Kernspinthomographen eingesetzt werden, weisen zweckmäßigerweise Markierungen auf, die aus einem Kernresonanz-Kontraststoff bestehen.

Gegebenenfalls kann es vorteilhaft sein, weitere Objekte mit den zuvor beschriebenen Markierungen zu versehen. Dabei kommen Bestandteile des verwendeten radiologischen Diagnosegerätes, wie z. B. der Patiententisch, aber auch der Patient selber in Frage. Knochen, innere Organe, Implantate etc. können mit Markierungen versehen werden, so dass deren räumliche Lage jederzeit mittels des erfindungsgemäßen Lokalisierungsverfahrens bestimmt werden kann. Der Operateur erhält somit die Möglichkeit die relative räumliche Anordnung der verwendeten Instrumente relativ zum Patienten jederzeit präzise zu bestimmen, ohne sich dafür auf präoperativ angefertigte Volumenaufnahmen des Patienten zurückbeziehen zu müssen.

Das erfindungsgemäße Verfahren kann, wie oben dargelegt, Vorteilhafterweise in Verbindung mit normalen, computergestützten radiologischen Diagnosegeräten zum Einsatz kommen, da lediglich ein Computerprogramm zur digitalen Bildverarbeitung benötigt wird, das aus den geometrischen Daten eines Objektes unter Berücksichtigung eines Kameramodells zweidimensionale Projektionsbilder berechnet und diese mit den radiologisch aufgenommenen Bilddaten vergleicht.

Ausführungsbeispiele der Erfindung werden im Folgenden anhand der Zeichnung näher erläutert. Es zeigen:
- Figur 1: ein C-Arm-Durchleuchtungsgerät zur Verwendung gemäß der Erfindung;
- Figur 2: erfindungsgemäße Markierung (schematisch);
- Figur 3: semitransparente Markierung in Kombination mit Röntgenopaken Elementen.

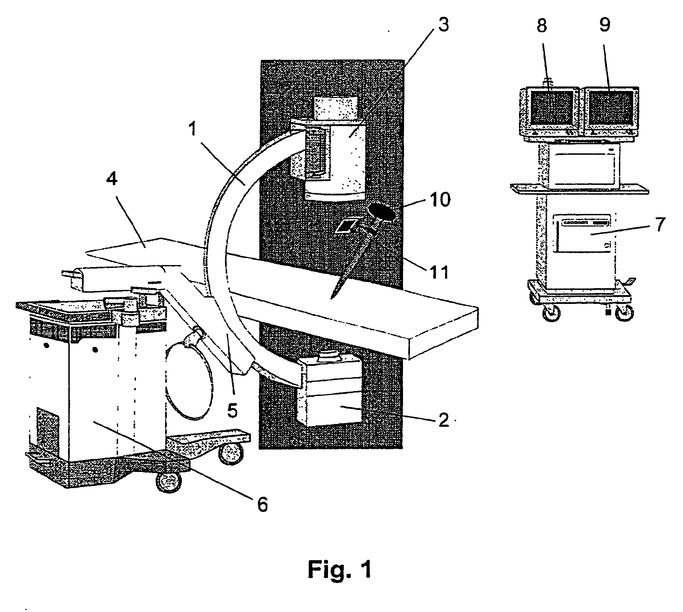

Ein C-Arm-Röntgengerät, wie es üblicherweise in der interventionellen Radiologie Verwendung findet, ist in der Figur 1 dargestellt. An dem C-Arm 1 sind eine Röntgenquelle 2 und ein gegenüberliegendes fluoroskopisches Bilderzeugungssystem 3 angeordnet. Der Patient, der sich auf einem Patiententisch 4 befindet, wird von einem konusförmigen Röntgenstrahlungsbündel durchleuchtet. Der C-Arm 1 ist in einer Führung 5 schwenkbar gelagert, so dass die Durchleuchtungsrichtung um eine parallel zum Patiententisch 4 verlaufende Achse gedreht werden kann. Der C-Arm ist an einem rollbaren Wagen 6 befestigt, der gleichzeitig Steuerungs- und Versorgungskomponenten des Röntgensystems aufnimmt. Das Röntgendurchleuchtungsgerät steht in Verbindung mit einem Bildverarbeitungs- und Visualisierungssystem, das aus einem Computer 7 zur digitalen Bildverarbeitung und zwei Monitoren 8 und 9 zur grafischen Darstellung und Anzeige der Röntgenprojektionen besteht. Ebenfalls dargestellt ist ein chirurgisches Instrument 10, das sich im durchleuchteten Volumenbereich oberhalb des Patiententisches 4 befindet. Die Position sowie die Orientierung des Instrumentes 10 lässt sich nach dem erfindungsgemäßen Verfahren mittels einer Markierung 11 bestimmen, die ebenfalls bei der Durchleuchtung als Projektion abgebildet wird. Die Markierung 11 ist mit dem chirurgischen Instrument 10 starr verbunden. Anhand des Projektionsbildes der Markierung 11 kann die räumliche Lage des chirurgischen Instrumentes 10, dessen geometrische Form bekannt ist, bestimmt werden und auf den Monitoren 8 und 9 in verschiedenen Ansichten derart dargestellt werden, dass der Operateur z. B. bei der Durchführung eines operativen Eingriffes einen räumlichen Eindruck von der Anordnung des chirurgischen Instrumentes 10 relativ zu dem auf dem Patiententisch 4 befindlichen Patienten erhält.

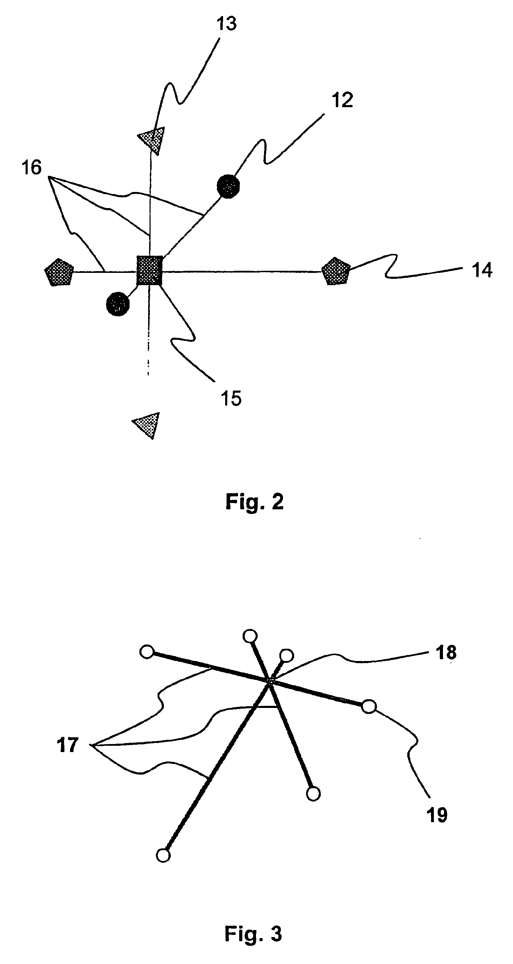

Die Figur 2 zeigt eine Markierung, die nach dem erfindungsgemäßen Verfahren dafür geeignet ist, aus einem Projektionsbild die Position und die Orientierung eines Objektes zu bestimmen. Die Markierung besteht aus Markierungskörpern 12, 13, 14, 15, die sich anhand ihrer geometrischen Form unterscheiden lassen. Durch zwei gegenüberliegende Markierungen gleicher Form werden jeweils Verbindungsachsen 16 definiert. Diese Verbindungsachsen schneiden sich in einem gemeinsamen Schnittpunkt, der durch den Markierungskörper 15 bezeichnet ist. Die drei Verbindungsachsen 16 werden durch den gemeinsamen Schnittpunkt 15 in Streckenabschnitte mit jeweils unterschiedlichen Längenverhältnissen aufgeteilt. Bei dem dargestellten Ausführungsbeispiel wird die zu den Markierungskörpern 12 gehörige Verbindungsachse etwa im Verhältnis 1 zu 3 geteilt, die zu den Markierungskörpern 13 gehörige Verbindungsachse etwa im Verhältnis 1 zu 1 und die zu den Markierungskörpern 14 gehörige Verbindungsachse etwa im Verhältnis 1 zu 2. Die drei Verbindungsachsen 16 sind orthogonal zueinander angeordnet. Aus einem Projektionsbild der dargestellten Markierung lässt sich nach dem erfindungsgemäßen Verfahren die Objektposition und -orientierung eindeutig bestimmen.

Die Figur 3 zeigt eine Markierung, die aus drei orthogonal angeordneten linienartigen Elementen 17 besteht. Diese bestehen aus einem Röntgen-semitransparenten Material, so dass durch die Markierung möglichst keine Bildinformationen des untersuchten Volumenbereiches verloren gehen. Die drei linienartigen Elemente 17 werden durch ihren gemeinsamen Schnittpunkt 18 wiederum in Streckenabschnitte mit unterschiedlichen Längenverhältnissen aufgeteilt. Die Lokalisierung nach dem erfindungsgemäßen Verfahren funktioniert zuverlässiger und effektiver, wenn die Markierungslinien 17 an ihren Enden zusätzlich mit Röntgen-opaken Elementen 19 versehen sind

## Patentansprüche

1. Verfahren zur Lokalisierung eines dreidimensionalen Objektes, insbesondere in der interventionellen Radiologie, bei dem ein Volumenbereich, in welchem sich das zu lokalisierende Objekt befindet, anhand von Projektionsbildern mittels digitaler Bildverarbeitung untersucht wird,
**dadurch gekennzeichnet,**
**dass** Projektionen des zu lokalisierenden Objektes, dessen geometrische Form bekannt ist, simuliert werden und mit dem Projektionsbild des untersuchten Volumenbereiches verglichen werden.

2. Verfahren nach Anspruch 1,
**dadurch gekennzeichnet,**
**dass** die Simulation so lange unter systematischer Variation der Parameter Objektposition und Objektorientierung wiederholt wird, bis eine optimale Übereinstimmung mit dem Projektionsbild besteht.

3. Verfahren nach Anspruch 1,
**dadurch gekennzeichnet,**
**dass** das zu lokalisierende Objekt mit wenigstens einer Markierung versehen ist, die auf dem Projektionsbild abgebildet wird.

4. Verfahren nach Anspruch 3,
**dadurch gekennzeichnet,**
**dass** die Markierung geometrisch derart gestaltet ist, dass sich ihrem zweidimensionalen Projektionsbild eindeutig die Position und die Orientierung der Markierung zuordnen lässt.

5. Verfahren nach Anspruch 3,
**dadurch gekennzeichnet,**
**dass** die Markierung aus linienartigen, sich schneidenden Strukturen besteht, die an ihrem Schnittpunkt in Abschnitte mit jeweils unterschiedlichen Längenverhältnissen unterteilt werden.

6. Verfahren nach Anspruch 1,
**dadurch gekennzeichnet,**
**dass** für die Simulation der Projektionen ein Volumenmodell des zu lokalisierenden Objektes verwendet wird, welches mittels dreidimensionaler Kernspinresonanz- oder Computerthomographie gewonnen wird.

7. Radiologisches Diagnosegerät, zur Durchführung des Verfahrens nach Anspruch 1, mit einem Patiententisch, einem digitalen Bilderzeugungssystem, einem Computer und wenigstens einem Anzeigegerät,
**dadurch gekennzeichnet,**
**dass** im Arbeitsspeicher des Computers ein gerätespezifisches, mathematisches Modell der Bildentstehung, geometrische Daten von zu lokalisierenden Objekten und ein Lokalisierungsprogramm abgelegt sind, das anhand der Geräte- und der Objektdaten die Parameter Objektposition und Objektorientierung relativ zum Patiententisch aus dem Projektionsbild ermittelt und auf dem Anzeigegerät visualisiert und wobei Projektionen des zu lokalisierenden Objektes, dessen geometrische Form bekannt ist, simuliert werden und mit dem projektionsbild des untersuchten Volumenbereichs verglichen werden.

8. Radiologisches Diagnosegerät nach Anspruch 7,
**dadurch gekennzeichnet,**
**dass** es sich dabei um ein Röntgendurchleuchtungsgerät, um einen Computertomographen oder um einen Kernspintomographen handelt.

9. Computer-Programmprodukt, das in der interventionellen Radiologie zur digitalen Bildverarbeitung eingesetzt wird, und wenn es auf einem Computer läuft oder in einem Computer geladen ist, den Computer veranlasst, ein Verfahren gemäß einem der Ansprüche 1-6 durchzuführen,
**dadurch gekennzeichnet,**
**dass** das Computerprogramm aus den geometrischen Daten eines Objektes unter Berücksichtigung eines Kameramodells zweidimensionale Projektionsbilder berechnet und diese mit den radiologisch aufgenommenen Bilddaten vergleicht.

## Claims

1. A method for localizing a three-dimensional object, particularly in interventional radiology, in which a volume area, in which the object which is to be localized is situated, is examined with the aid of projection images by means of digital image processing,
**characterized in that**
projections of the object which is to be localized, the geometric shape of which is known, are simulated and are compared with the projection image of the examined volume area.

2. The method according to claim 1,
**characterized in that**
the simulation is repeated whilst systematically varying the parameters concerning the position and orientation of the object, until an optimum correspondence with the projection image exists.

3. The method according to claim 1,
**characterized in that**
the object which is to be localized is provided with at least one marker which is reproduced on the projection image.

4. The method according to claim 3,
**characterized in that**
the marker is geometrically arranged such that the position and the orientation of the marker can be unambiguously correlated with its two-dimensional projection image.

5. The method according to claim 3,
**characterized in that**
the marker comprises line-like, intersecting structures which are subdivided into segments with different respective length ratios at their point of intersection.

6. The method according to claim 1,
**characterized in that**
a volume model of the object which is to be localized is used for the simulation of the projections, said volume model being obtained by means of three-dimensional magnetic resonance tomography or computed tomography.

7. A radiological diagnostic apparatus to carry out the method according to claim 1, having a patient table, a digital imaging system, a computer and at least one display device,
**characterized in that**
an apparatus-specific, mathematical model of the imaging, geometrical data of objects which are to be localized and a localization programme are stored in the working memory of the computer, said programme determining the parameters concerning the position and orientation of the object relative to the patient table on the basis of the apparatus data and object data, and visualizing these on the display device, and in which projections of the object which are to be localized, the geometric shape of which is known, are simulated and are compared with the projection image of the examined volume area.

8. The radiological diagnostic apparatus according to claim 7,
**characterized in that**
it is a radioscopy apparatus, a computer tomography apparatus or a magnetic resonance tomography apparatus.

9. A computer programme product which is used for digital image processing in interventional radiology and, when it runs on a computer or is loaded into a computer, causes the computer to carry out a method according to any of claims 1 to 6,
**characterized in that**
the computer programme calculates two-dimensional projection images from the geometric data of an object, taking into account a camera model, and compares these with the radiologically recorded image data.

## Revendications

1. Procédé de localisation d'un objet tridimensionnel, notamment dans la radiologie interventionnelle, comportant à analyser une zone volumique dans laquelle se trouve l'objet à localiser, à l'aide d'images de projection, au moyen d'un traitement numérique des images, **caractérisé en ce qu'**on simule des projections de l'objet à localiser, dont la forme géométrique est connue et qu'on les compare avec l'image de projection de la zone volumique analysée.

2. Procédé selon la revendication 1, **caractérisé en ce qu'**on répète la simulation sous variation systématique des paramètres "position de l'objet" et "orientation de l'objet", jusqu'à obtenir une concordance optimale avec l'image de projection.

3. Procédé selon la revendication 1, **caractérisé en ce que** l'objet à localiser est muni d'au moins un marquage, que l'on reproduit sur l'image de projection.

4. Procédé selon la revendication 3, **caractérisé en ce que** le marquage a une conception géométrique telle, qu'il est possible d'associer indubitablement la position et l'orientation du marquage à son image de projection bidimensionnelle.

5. Procédé selon la revendication 3, **caractérisé en ce que** le marquage comporte dans des structures qui se recoupent, du type de lignes, que l'on divise sur leur point d'intersection en tronçons avec chaque fois différents rapports de longueur.

6. Procédé selon la revendication 1, **caractérisé en ce que** pour la simulation des projections, on utilise un modèle volumique de l'objet à localiser, que l'on obtient au moyen d'une tomographie à résonance magnétique nucléaire ou d'une tomographie informatique.

7. Appareil de diagnostic radiologique, pour réaliser le procédé selon la revendication 1, avec une table de soin, un système numérique de création d'image, un ordinateur et au moins un instrument d'affichage, **caractérisé en ce que** dans la mémoire centrale de l'ordinateur sont sauvegardés un modèle mathématique spécifique à l'appareil pour la formation de l'image, des informations géométriques concernant des objets à localiser et un programme de localisation, qui à l'aide des informations concernant l'appareil et l'objet détermine les paramètres "position de l'objet" et "orientation de l'objet" vis à vis la table de soin et les visualise sur l'instrument d'affichage, et des projections de l'objet à localiser, dont la forme géométrique est connue étant simulées et comparées avec l'image de projection de la zone volumique analysée.

8. Appareil de diagnostic radiologique selon la revendication 7, **caractérisé en ce qu'**il s'agit à cet effet d'un radioscope, d'un tomographe informatique ou d'un tomographe à résonance magnétique.

9. Programme informatique, utilisé dans la radiologie interventionnelle pour le traitement numérique des images et qui, lorsqu'il s'exécute sur un ordinateur ou lorsqu'il est chargé dans un ordinateur incite l'ordinateur à réaliser un procédé selon l'une quelconque des revendications 1 à 6, **caractérisé en ce que** le programme informatique calcule à partir des informations géométriques d'un objet, sous considération d'un modèle de caméra, des images de projection bidimensionnelles et les compare avec les informations image relevées par radiologie.
